# EUROPEAN PATENT APPLICATION

(11) **EP 3 799 798 A1**
(43) Date of publication of application: **07.04.2021**
(21) Application number: 18927445.9
(22) Date of filing: 27.07.2018
(51) Int. Cl.: A61B 17/00

(54) **TRANSPLANTATION TENDON EXTRACTOR**

(71) Applicant: Public University Corporation Nara Medical University, Nara 634-8521 (JP); Nara Seiko Inc., Sakurai-shi, Nara 633-0101 (JP)
(72) Inventor: NAKANISHI, Yasuaki, Kashihara-shi, Nara 634-8521 (JP); OMOKAWA, Shohei, Kashihara-shi, Nara 634-8521 (JP); NAKAGAWA, Hiroo, Sakurai-shi, Nara 633-0101 (JP)
(74) Representative: Manitz Finsterwald Patent- und Rechtsanwaltspartnerschaft mbB
(86) International application number: PCT/JP2018/028210
(87) International publication number: WO 2020/021695

(57) **Abstract**

To provide a transplant-use tendon harvesting device that can harvest a tendon more easily.

The transplant-use tendon harvesting device 1 includes a mantle portion 5 and a tendon separation element 8. The mantle portion 5 is configured to be inserted into a body from an incision portion 4 and moved along a tendon 3 to be harvested. The tendon separation element 8 is formed of an elastic member and attached to the mantle portion 5 to move along the tendon 3 together with the mantle portion 5 so as to separate an end portion of the tendon 3 on the moving direction side from another tissue. The tendon separation element 8 includes a separation functional portion 6 to cause the tendon 3 to pass inside at a time of moving along the tendon 3 to separate the tendon 3 and linear portions 11 and 12 inserted into the mantle portion 5 to be fixed.

## Description

### TECHNICAL FIELD

The present invention relates to a surgical device used for harvesting tendon graft for musculoskeletal surgery.

### BACKGROUND

Conventionally, there is known a medical apparatus used for harvesting a tendon for transplantation in surgeries by ablation or separation (for example, see Patent Document 1). The medical apparatus in Patent Document 1 includes a cannulated rod member to be inserted into a body and a tendon separating loop wire inserted in the rod member. The separating loop wire has a dissection part for separating the tendon from muscle tissue at its proximal end.

When we harvest the tendon, we make a small incision of skin, where the distal end of tendon is identified under the subcutaneous soft tissue. Next, the tendon is trapped with loop portion of the separating loop wire. Then, the loop wire is inserted in the rod member. Thus, the tendon is trapped in the loop, where is located at the end of the rod member.

Next, the rod member is inserted into the body along the tendon from distal to proximal. When the rod member is pushed toward proximal direction in the muscle around the tendon, the muscle fibers attaching the tendon are separated from the surface of the tendon by the loop wire. After the loop reached at the proximal end of the tendon, the tendon is totally separated from the muscle body. Thus, the cut-off tendon is extracted from inside of the body via the incision portion.

Patent Document 1: WO 2008/093747

### DISCLOSURE OF THE INVENTION

### PROBLEMS TO BE SOLVED BY THE INVENTION

However, since the above-described medical apparatus incudes the separation means having the cutting part at the distal end portion of the rod member, the medical apparatus needs a careful manipulation so as not to damage the inside of the body with the separation means and/or the cutting part when the rod member is inserted into the body along the tendon. In view of this, a surgeon is required to have proficiency in handling the medical apparatus and the like to some extent.

The object of the present invention is to provide a transplant-use tendon harvesting device that can harvest the tendon more easily in consideration of the problem of the prior art.

### SOLUTIONS TO THE PROBLEMS

A transplant-use tendon harvesting device of the present invention includes:
an elongate mantle portion configured to be inserted into a body from an incision portion and moved in a longitudinal direction along a tendon to be extracted or harvested; and
a tendon separation element that is formed of an elongate elastic member and attached to the mantle portion to move along the tendon together with the mantle portion so as to separate an end portion of the tendon on the moving direction side from other tissues,
the tendon separation element includes:
   an annular separation functional portion that is formed by a middle portion of the elastic member to cause the tendon to pass inside at a time of moving along the tendon to separate the tendon; and
   linear portions on both sides of the separation functional portion configured to be fixed by being inserted into the mantle portion.

According to the present invention, when the tendon is harvested, the tendon separation element is first arranged such that the separation functional portion of the tendon separation element has the tendon positioned in its inside through the incision portion. At this time, the tendon separation element has elasticity, and thus, by mutually opening the linear portions on its both sides and passing one of the linear portions through a lower side of the tendon, the tendon can be easily arranged inside the separation functional portion.

Next, the linear portions on both the sides of the tendon separation element are inserted into the mantle portion and fixed to the mantle portion. Then, the mantle portion is inserted into the body along the tendon. Since this causes the tendon separation element to also move to an inserting direction, the tendon passes inside the separation functional portion.

Then, when the separation functional portion reaches the end portion on the moving direction side of the tendon, the tendon to be extracted or harvested is separated so as to be scraped off from a muscle with the separation functional portion. Afterwards, the tendon in which the end portion is separated is pulled outside the body from the incision portion to be cut off at an end portion on its opposite side, and the tendon can be harvested.

Accordingly, a surgeon does not need proficiency in manipulating the tendon separation element and the mantle portion, and can easily extract or harvest the tendon. Additionally, since the separation functional portion that separates the tendon is configured in a middle portion of the tendon separation element formed of the elastic member, even an inexperienced surgeon can easily insert the mantle portion equipped with the tendon separation element into the body and separate the tendon without a risk of causing a damage to the tendon and tissues in its peripheral portion.

In the present invention, the mantle portion is preferably configured as follows. The mantle portion has an inserted portion as a portion to be inserted into the body. The inserted portion has a lateral cross-sectional surface in an elliptical shape. The linear portions on both the sides are arranged side by side in a long axis direction of the elliptical shape in a state where the linear portions are inserted into the mantle portion to be fixed.

With this, since the mantle portion has the lateral cross-sectional surface in the elliptical shape and the linear portions on both the sides of the tendon separation element are aligned in the long axis direction of the elliptical shape of the mantle portion, an area of the lateral cross-sectional surface can be reduced compared with a case of having a lateral cross-sectional surface in a circular shape. This ensures inserting the mantle portion into the body more smoothly while avoiding interference with surrounding tissues as much as possible.

In the present invention, an opening inside the annular separation functional portion preferably does not overlap the inserted portion as viewed along a longitudinal direction of the mantle portion, and has an elliptical shape having a short axis positioned on a straight line passing through a short axis of the elliptical shape of the inserted portion.

With this, the shape of the separation functional portion can be easily adapted to the cross-sectional shape of the tendon having the elliptical shape, and the size of the separation functional portion can be minimally set. Furthermore, since the short axes of the elliptical shapes of the opening of the separation functional portion and the mantle portion are positioned on the same straight line, the mantle portion can be inserted into the body more smoothly.

In the present invention, the mantle portion is preferably provided with a fixing portion that fixes the tendon separation element in a releasable manner to the mantle portion on a rear end side with respect to the inserted portion.

With this, the tendon separation element is fixed to the mantle portion by the fixing portion, and thus, the inserted portion of the mantle portion can be inserted into the body and pulled out from the inside of the body together with the tendon separation element.

In the present invention, the elastic member is preferably an elastic wire. This facilitates the formation of the tendon separation element.

### BRIEF DESCRIPTION OF THE DRAWINGS

FIG. 1A to FIG. 1D are partial cross-sectional views illustrating a state where a transplant-use tendon harvesting device according to one embodiment of the present invention is used.
FIG. 2A and FIG. 2B are a front view and a side view of the transplant-use tendon harvesting device in FIG. 1 in a state where a tendon separation element is attached to a mantle portion.
FIG. 3A and FIG. 3B are a front view and a side view of the mantle portion of the transplant-use tendon harvesting device in FIG. 2.
FIG. 4 is a perspective view of a fixing block in a fixing portion of the transplant-use tendon harvesting device in FIG. 2.
FIG. 5 is a front view of a fixing screw in the fixing portion of the transplant-use tendon harvesting device in FIG. 2.

### DESCRIPTION OF PREFERRED EMBODIMENTS

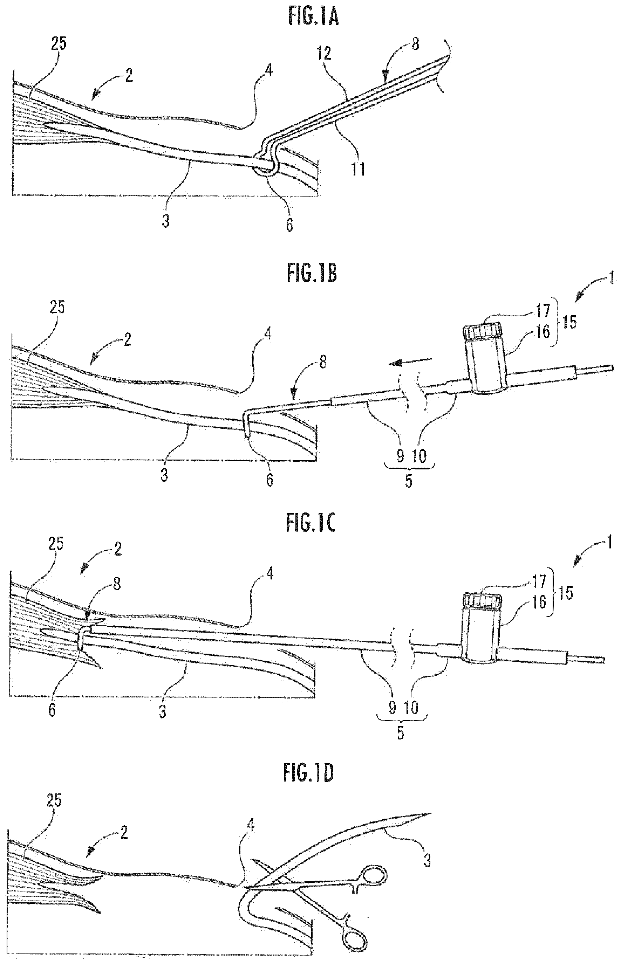

The following describes an embodiment of the present invention using the drawings. FIG. 1A to FIG. 1D illustrate a state where a transplant-use tendon harvesting device according to one embodiment of the present invention is used. As illustrated in FIG. 1, this transplant-use tendon harvesting device 1 is used for extracting a tendon 3 of a human body 2.

The transplant-use tendon harvesting device 1 includes an elongate mantle portion 5 and an annular separation functional portion 6. The mantle portion 5 is inserted into the body from an incision portion 4 and moved along the tendon 3. The separation functional portion 6 causes the tendon 3 to pass its inside to separate the tendon 3 when the mantle portion 5 moves along the tendon 3. The separation functional portion 6 is formed as a part of a tendon separation element 8.

The tendon separation element 8 is formed of an elastic wire as an elongate elastic member. The elastic wire preferably has elasticity enough to maintain a shape similar to a shape when the tendon separation element 8 is attached to the mantle portion 5 without applying a force.

The tendon separation element 8 is attached to the mantle portion 5, and moves along the tendon 3 in a longitudinal direction together with the mantle portion 5. This causes an end portion of the tendon 3 on this moving direction side to be separated from other tissues.

As the tendon 3 to be harvested, for example, a palmaris longus muscle tendon, a plantaris muscle tendon, and an abductor pollicis longus muscle tendon are considered. These tendons are usually unlikely to affect functionalities of the human body even when the tendons are harvested. For example, these tendons can be used as an autogenous tendon that is transplanted to a patient having impairment of finger functions.

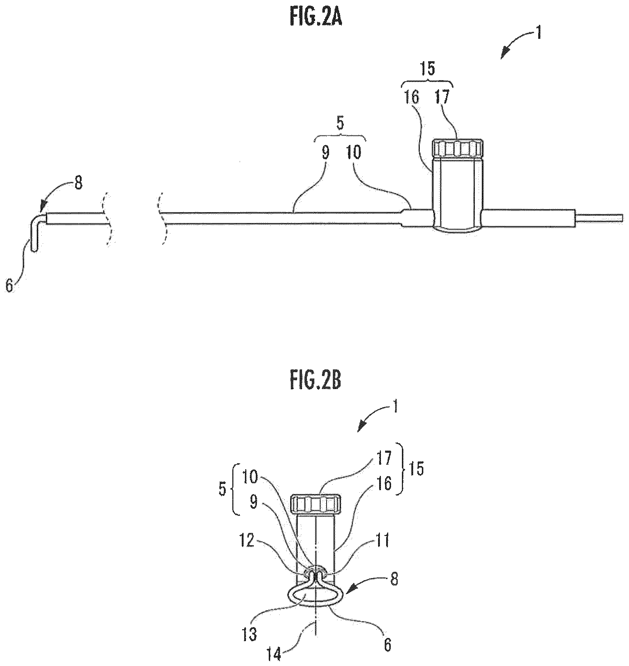

FIG. 2A and FIG. 2B illustrate the transplant-use tendon harvesting device 1 in a state where the tendon separation element 8 is attached to the mantle portion 5. As illustrated in FIG. 2A and FIG. 2B, the separation functional portion 6 is configured by a folded portion of the elastic wire constituting the tendon separation element 8. That is, the separation functional portion 6 is formed by cutting a wire rod with a diameter of approximately l mm to a length corresponding to an overall length of the elastic wire constituting the tendon separation element 8, and shaping its middle portion in an elliptical shape.

The mantle portion 5 has an inserted portion 9 as a portion to be inserted into the body, and the inserted portion 9 has a lateral cross-sectional surface in an elliptical shape. In the mantle portion 5, a mantle rear end portion 10 adjacent to a rear end side of the inserted portion 9 has a lateral cross-sectional surface in a circular shape. Inside the mantle portion 5, linear portions 11 and 12 on both sides of the separation functional portion 6 as the folded portion of the elastic wire are arranged side by side in a long axis direction of the elliptical shape.

The separation functional portion 6 has an opening 13 through which the tendon 3 passes when the inserted portion 9 of the mantle portion 5 is inserted into the body. This opening 13 does not overlap the mantle portion 5 as viewed along a longitudinal direction of the mantle portion 5, and has an elliptical shape having a short axis positioned on a straight line 14 passing through a short axis of the elliptical shape of the inserted portion 9. That is, the short axes of the elliptical shapes of the opening 13 of the separation functional portion 6 and the mantle portion 5 are positioned on the same straight line 14.

The mantle portion 5 is provided with a fixing portion 15 that fixes the wire 8 in a releasable manner to the mantle portion 5 on the rear end side with respect to the inserted portion 9, that is, on the mantle rear end portion 10. The fixing portion 15 includes a fixing block 16 and a fixing screw 17 screwed to the fixing block 16.

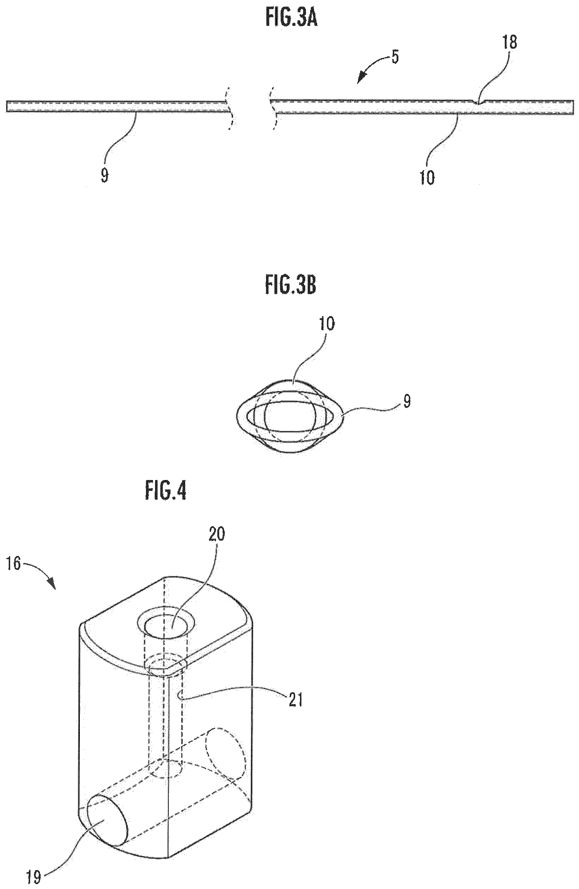

FIG. 3A and FIG. 3B illustrate the mantle portion 5. As illustrated in FIG. 3A and FIG. 3B, a portion on the mantle rear end portion 10 where the fixing portion 15 is disposed is provided with a through hole 18 reaching from a side surface to an inner surface of the mantle rear end portion 10. The fixing portion 15 and the through hole 18 are configured such that a distal end portion of the fixing screw 17 projects from the inner surface. A center axis line of the through hole 18 is parallel to the above-described straight line 14.

A diameter of the mantle rear end portion 10 is, for example, 2.7 mm. The mantle portion 5 can be formed by, for example, cutting a tubular member with a diameter of the cross-sectional surface of 2.7 mm and a thickness of 0.35 mm to the length of the mantle portion 5 of, for example, about 300 mm, crushing a portion corresponding to the inserted portion 9 to be shaped such that the cross-sectional surface becomes to have the elliptical shape, and providing the through hole 18.

FIG. 4 illustrates the fixing block 16. As illustrated in FIG. 4, the fixing block 16 is provided with a penetrating insertion hole 19 and a screw hole 20. The mantle rear end portion 10 of the mantle portion 5 is inserted into the insertion hole 19 to be fixed. The screw hole 20 is communicated with the insertion hole 19 from a top end surface of the fixing block 16 so as to be perpendicular to the insertion hole 19. The screw hole 20 is provided with a female screw 21 with which the fixing screw 17 is screwed. A center axis line of the screw hole 20 is parallel to the above-described straight line 14.

The fixing block 16 is positioned with respect to the mantle rear end portion 10 of the mantle portion 5 such that the center axis line of the screw hole 20 corresponds to the center axis line of the through hole 18 of the mantle rear end portion 10, and fixed by welding. For the welding, a laser welding machine and the like can be used.

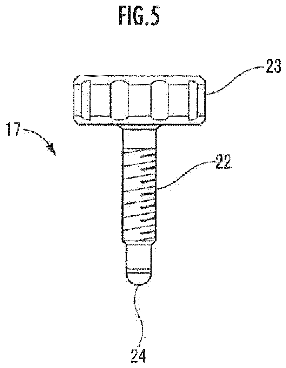

FIG. 5 illustrates the fixing screw 17. As illustrated in FIG. 5, the fixing screw 17 includes a male screw 22 screwed with the female screw 21 of the fixing block 16 and a knob 23 for screwing the male screw 22 with the female screw 21.

A distal end portion of the male screw 22 is provided with a projecting portion for fixing 24 that projects to the inner surface of the mantle portion 5 via the through hole 18 of the mantle portion 5 by screwing with the female screw 21 to fix the wire 8 to the mantle portion 5. A projection side end portion of the projecting portion for fixing 24 has a hemispherical shape.

When the tendon 3 of the human body 2 is harvested using the transplant-use tendon harvesting device 1, the incision portion 4 is preliminarily formed at a position of the human body 2 suitable for extracting or harvesting the intended tendon 3, and the tendon separation element 8 is arranged such that the tendon 3 is positioned inside the separation functional portion 6 of the tendon separation element 8 through the incision portion 4 as in FIG. 1A.

At this time, the tendon separation element 8 has elasticity, and thus, by opening its linear portions 11 and 12 and passing the linear portion 11 on one side through a lower side of the tendon 3, the tendon 3 can be easily arranged inside the separation functional portion 6.

Next, as in FIG. 1B, the linear portions 11 and 12 on both the sides of the tendon separation element 8 are inserted into the mantle portion 5 and fixed to the mantle portion 5 with the fixing screw 17. Then, as in FIG. 1C, the mantle portion 5 is inserted into the body along the tendon 3. Since this causes the tendon separation element 8 to also move in an inserting direction, the tendon 3 passes inside the separation functional portion 6.

Then, when the separation functional portion 6 reaches the end portion of the tendon 3 on the moving direction side, the end portion is separated so as to be scraped off from the other tissues, such as a muscle 25, with the separation functional portion 6. That is, a surgeon can separate the tendon 3 from the muscle 25 by simply inserting the mantle portion 5 along the tendon 3. At that time, since the separation functional portion 6 is formed by the folded portion of the elastic wire, there is no risk of damage on the tissues inside the body.

Afterwards, the transplant-use tendon harvesting device 1 is pulled out from the inside of the body. Then, as in FIG. 1D, the tendon 3 in which the end portion on the muscle 25 side is separated is pulled outside the body from the incision portion 4 to be cut off at an end portion on its opposite side, and the extraction of the tendon 3 is completed.

The tendon 3 harvested in this manner can be used, for example, as the autogenous transplant-use tendon, for a tendon reconstruction operation for a patient who has the impairment of finger functions and thumb carpometacarpal joint arthrosis due to injuries and rheumatoid arthritis.

As described above, with this embodiment, since the separation functional portion 6 is configured by the folded portion of the elastic wire, the tendon 3 can be separated from the muscle and the like by simply inserting the mantle portion 5 along the tendon 3 after the tendon separation element 8 is attached to the mantle portion 5. At that time, the mantle portion 5 can be easily inserted into the body without causing a damage to the tendon 3 or the other tissues inside the body.

Conventionally, when the palmaris longus muscle tendon, the plantaris muscle tendon, and the abductor pollicis longus muscle tendon are harvested, the extraction has been performed by touch after making incisions at several positions. By comparison, this embodiment can shorten a surgery time, reduce a burden on patients and doctors, and limit a scar of the incision portion to one position.

Additionally, since the mantle portion 5 has the inserted portion 9 having the lateral cross-sectional surface in the elliptical shape, interference with the tissues inside the body is minimalized compared with a case where the lateral cross-sectional surface has a circular shape, and a smooth insertion inside the body can be ensured.

Additionally, since the opening 13 of the separation functional portion 6 has the elliptical shape, the shape of the separation functional portion 6 is easily adapted to a cross-sectional shape of the tendon 3 having an elliptical shape to ensure setting a size of the separation functional portion 6 to minimum. Furthermore, the opening 13 does not overlap the mantle portion 5 as viewed along the longitudinal direction of the mantle portion 5 and has the short axis positioned on the straight line 14 passing through the short axis of the elliptical shape of the mantle portion 5. Thus, the mantle portion 5 can be inserted into the body more smoothly.

Note that the present invention can be carried out in its range without being limited to the above-described embodiment. For example, the tendon 3 to be harvested is not limited to the above-described palmaris longus muscle tendon and the like, but may be another tendon. Furthermore, the tendon 3 to be harvested is not limited to the tendon in the human body, but may be a tendon in domestic animals. Additionally, a portion of the tendon 3 to be separated by the separation functional portion 6 may be an end portion of a tendon insertion site side on the opposite side from a muscle.

Additionally, the mantle portion 5 and the tendon separation element 8 can be formed of a titanium alloy or a stainless steel alloy, but this should not be construed in a limiting sense. The mantle portion 5 and the tendon separation element 8 may be formed of another metal, synthetic resin, and the like.

Additionally, a cross-sectional shape of the inserted portion 9 is not limited to the elliptical shape, but may be another shape, for example, a rectangular shape having an arc-shaped short side, as long as the cross-sectional shape has a shape as flat as possible that can be inserted without resistance and causes no damage to the tendon 3 and other portions.

Additionally, the tendon 3 to be harvested has various lengths, such as 15 to 17 cm for the palmaris longus muscle tendon, 20 to 25 cm for the plantaris muscle tendon, and 6 to 7 cm for the abductor pollicis longus muscle tendon, and the length also varies slightly depending on physical size difference. Accordingly, a length of the inserted portion 9 is preferably around 30 cm so as to correspond to these lengths. However, if a kind of the tendon 3 to be harvested is determined, the inserted portion 9 may have the length exclusive for the determined tendon 3.

Additionally, several kinds of the tendon separation elements 8 with the separation functional portions 6 being different in size may be prepared, and the transplant-use tendon harvesting device 1 may be used by exchanging the tendon separation elements 8 as necessary corresponding to a portion and a size of the tendon 3 to be harvested and a physical size of a human having the tendon 3.

### DESCRIPTION OF REFERENCE SIGNS

- 1: Transplant-use tendon harvesting device
- 2: Human body
- 3: Tendon
- 4: Incision portion
- 5: Mantle portion
- 6: Separation functional portion
- 8: Tendon separation element
- 9: Inserted portion
- 10: Mantle rear end portion
- 11, 12: Linear portion
- 13: Opening
- 14: Straight line
- 15: Fixing portion
- 16: Fixing block
- 17: Fixing screw
- 18: Through hole
- 19: Insertion hole
- 20: Screw hole
- 21: Female screw
- 22: Male screw
- 23: Knob
- 24: Projecting portion for fixing

## Claims

1. A transplant-use tendon harvesting device comprising:
an elongate mantle portion configured to be inserted into a body from an incision portion and moved in a longitudinal direction along a tendon to be harvested; and
a tendon separation element that is formed of an elongate elastic member and attached to the mantle portion to move along the tendon together with the mantle portion so as to separate an end portion of the tendon on the moving direction side from other tissues, wherein
the tendon separation element includes:
an annular separation functional portion that is formed by a middle portion of the elastic member to cause the tendon to pass inside at a time of moving along the tendon to separate the tendon; and
linear portions on both sides of the separation functional portion configured to be fixed by being inserted into the mantle portion.

2. The transplant-use tendon harvesting device according to claim 1, wherein
the mantle portion has an inserted portion as a portion to be inserted into a body, the inserted portion having a lateral cross-sectional surface in an elliptical shape, the linear portions on both the sides being arranged side by side in a long axis direction of the elliptical shape in a state where the linear portions are inserted into the mantle portion to be fixed.

3. The transplant-use tendon harvesting device according to claim 2, wherein
an opening inside the annular separation functional portion does not overlap the inserted portion as viewed along a longitudinal direction of the mantle portion, and has an elliptical shape having a short axis positioned on a straight line passing through a short axis of the elliptical shape of the inserted portion.

4. The transplant-use tendon harvesting device according to claim 1, wherein
the mantle portion is provided with a fixing portion that fixes the tendon separation element in a releasable manner to the mantle portion on a rear end side with respect to the inserted portion.

5. The transplant-use tendon harvesting device according to claim 1, wherein
the elastic member is an elastic wire.
